# EUROPÄISCHE PATENTANMELDUNG

(11) **EP 0 939 072 A1**
(43) Veröffentlichungstag der Anmeldung: **01.09.1999**
(21) Anmeldenummer: 99102930.7
(22) Anmeldetag: 13.02.1999
(51) Int. Cl.: C07C 53/06

(54) **Stückiges Calciumformiat**

(30) Priorität: 26.02.1998 DE 19807996
(71) Anmelder: Bayer Aktiengesellschaft, 51368 Leverkusen (DE)
(72) Erfinder: Noack, Achim, 42799 Leichlingen (DE); Baumgartner, Hanspeter, Dr., 47807 Krefeld (DE); Linde, Günter, Dr., 47800 Krefeld (DE); Klausener, Alexander, Dr., 50259 Pulheim (DE)

(57) **Zusammenfassung**

Stückige Materialien, die 5-100 Massen-% Calciumformiat und 95-0 Massen-% Zusatzstoffe enthalten und Partikelgrößen von 0,2-5 mm aufweisen, können aus feinkristallinem Calciumformiat und den Zusatzstoffen durch Verdichtung und, falls erforderlich, nachfolgende Zerkleinerung und/oder Fraktionierung zur Einstellung der Partikelgröße hergestellt werden.

## Beschreibung

Der Gegenstand der Erfindung ist ein Verfahren zur Herstellung eines stückigen, Calciumformiat der Formel Ca(HCOO)₂ enthaltenden Materials, wobei man feinkörniges Calciumformiat, gegebenenfalls unter Zusatz eines oder mehrerer Hilfsstoffe sowie gegebenenfalls unter Beimischung einer oder mehrerer weiterer anwendungsfördernder Komponenten, einer Verdichtung unterwift und gegebenenfalls das so erhaltene Material in der Folge einer Zerkleinerung und/oder Fraktionierung zwecks Einstellung definierter Partikelgrößen zuführt. Ein weiterer Gegenstand der Erfindung ist verdichtetes, Calciumformiat enthaltendes Material mit einem Korngrößenbereich von 0,2-5 mm.

Calciumformiat ist ein heller, kristalliner Feststoff, der beispielsweise in folgenden Bereichen kommerziell eingesetzt wird:
- Zusatzstoff im Bereich der Tierernährung (Schweine-, Rinder- und Putenmast)
- Einsatz im Bereich der Baustoffindustrie (Verbesserung der Aushärtung von Zement, Gips und Fugenmassen sowie Frostschutzmittel für Mörtel)
- Herstellung von Ameisensäure
- Hilfsstoff in der Lederindustrie
- Hilfsmittel bei der Herstellung von Hochglanzpapieren
- Behandlung von Waschwässern bei der Rauchgasentschwefelung
- Hilfsmittel bei der Silierung.

Man erhält Calciumformiat auf verschiedene Weisen, beispielsweise durch Umsetzung von Ameisensäure mit Calciumhydroxid oder durch Einwirkung von Kohlenmonoxid auf Calciumhydroxid. Derartige Synthesen sind beispielsweise in Ullmann's Enzyklopädie der Organischen Chemie (4. Auflage), Bd. 7, S. 366 und 370 beschrieben.

Die technisch bedeutungsvollste Gewinnung von Calciumformiat erfolgt innerhalb verschiedener literaturbekannter Verfahren zur Herstellung von Polyolen, bei denen Calciumformiat als Nebenprodukt erhalten wird (vgl. Ullmann, 5. Aufl., Bd. A 12, S. 29). Beispielhaft genannt sei hier die Herstellung von Trimethylolpropan aus n-Butyraldehyd und Formaldehyd unter Verwendung von Calciumhydroxid als Base. Bei diesem Prozeß fällt Calciumformiat als Koppelprodukt an.

Calciumformiat läßt sich mit Hilfe grundsätzlich bekannter Techniken kristallisieren, isolieren und reinigen. Als Beispiele für dabei einsetzbare Kristallisationstechniken seien beispielhaft die Verdampfungskristallisation wäßriger, gegebenenfalls organisches Material enthaltender Lösungen, die Kühlkristallisation und die Sprühtrocknung genannt. Als Beispiele für geeignete Isolierungstechniken seien beispielsweise das Filtrieren oder das Zentrifugieren genannt. Als mögliche Reinigungsoperationen seien beispielhaft Umlösung, Dispergierung, Waschung und Extraktion mit geeigneten Lösungsmitteln genannt. Gegebenenfalls schließen sich derartigen Operationen bzw. Operationssequenzen ein oder mehrere geeignete Trocknungsschritte an, von denen als geeignete Verfahren beispielhaft die Heißlufttrocknung, die Kontakttrocknung und die Vakuumtrocknung genannt seien.

Allen diesen Prozessen ist gemein, daß sie polydisperse Produkte liefern, die durch charakteristische, mehr oder weniger breite Korngrößenverteilungen gekennzeichnet sind. Bei allen bekannten technischen Verfahren zur Herstellung von Calciumformiat finden sind insbesondere im feinkörnigen Bereich mit Partikeldurchmessern <0,2 mm signifikant hohe Anteile des Gesamtkristallisates. Für verschiedene Einsatzzwecke benötigt man jedoch vorzugsweise ein Produkt, das einen besonders hohen Anteil an grobkörnigen, möglichst stückigen Partikeln aufweist. Gründe hierfür können beispielsweise in bestimmten Forderungen der Arbeitshygiene liegen, denen zufolge der Umgang mit staubförmige Anteile enthaltendem Calciumformiat Probleme bereiten kann, die sich nur mit zusätzlichen, wirtschaftliche Nachteile verursachenden technischen Maßnahmen beheben lassen. Andere Gründe sind auf das physikalische Verhalten von feinkörnigem Calciumformiat zurückzuführen. Bringt man beispielsweise in bestimmte handelsübliche grobkörnige, strukturierte oder geformte Tierfutter- oder Silierkomponenten feinkörniges oder hohe Feinkornanteile enthaltendes Calciumformiat ein, so ist dies oftmals mit erheblichen Mischungsproblemen sowie bei der weiteren Handhabung des fertigen Gemisches mit unerwünschten Entmischungen verbunden. Diese Effekte können den an sich vorteilhaften Einsatz von Calciumformiat in vielen Anwendungsbereichen begrenzen. Zwar gelingt es, mit Hilfe geeigneter Techniken wie Klassieren, Sieben und Sichten grobkörnige Anteile zu separieren, doch werden einerseits hierdurch zusätzliche Kosten verursacht, andererseits können die dabei zurückbleibenden feinkörnigen Anteile nur unter erheblichem zusätzlichen Aufwand einer weiteren Nutzung zugeführt werden, indem sie beispielsweise erneut aufgelöst, kristallisiert, isoliert, getrocknet und abermals dann dem Fraktionierungsprozeß zugeführt werden. Gerade in Prozessen, bei denen Calciumformiat lediglich als Nebenprodukt erhalten wird, ist es oftmals technisch und ökonomisch nicht sinnvoll, die Isolierung des bevorzugt angestrebten Hauptproduktes so zu modifizieren, daß Calciumformiat in möglichst grobkörnigen Fraktionen anfällt.

Zur Überführung von feinkörnigen oder pulverförmigen Feststoffen in stückiges Material stehen grundsätzlich verschiedene Techniken der Agglomeration zur Verfügung, deren Anwendung grundsätzlich auch im Falle von kristallinem Calciumformiat erwogen werden könnten. Durchgeführte Versuche, Calciumformiat beispielsweise durch Zusatz geringer Mengen Wasser so zu befeuchten, daß, basierend auf einem Zusammenkleben feiner Partikel größere Kristallverbände entstehen, führen jedoch aufgrund der hydroskopischen Eigenschaften von trockenem Calciumformiat im allgemeinen zur Bildung undefinierter Klumpen neben unverändert feinkörnigem Material. Aufgrund des äußerst spröden Verhaltens von kristallinem Calciumformiat sind weiterhin formgebende Verfahren zur Verdichtung nicht ohne weiteres erfolgreich, da die dabei anfallenden Partikel leicht zum Zerbröckeln neigen und hierdurch unerwünschter Feinkornanteil generiert wird. Versuche, diesen Effekt durch Hinzufügen von Bindemittel zurückzudrängen, scheitern schon an den unterschiedlichen anwendungstechnischen Forderungen, die aufgrund seines vielfältigen Einsatzes an Calciumformiat gestellt werden und die häufig nicht die Verunreinigung durch weitere Zusatzstoffe erlauben.

Im Hinblick auf die Herstellung vor allem verschiedener Tierfutterprodukte und Silierhilfsmittel bestand daher die Aufgabe, auf Basis von Calciumformiat ein grobkörniges, möglichst stückiges Material herzustellen, das den Anforderungen gerecht wird, eine möglichst enge und daher gut zu verarbeitende Partikelgrößenverteilung bei gleichzeitig möglichst geringem Feinkornanteil aufzuweisen. Weiterhin bestand die Aufgabe, einen technisch gangbaren, wirtschaftlichen Weg zur Herstellung eines derartigen Produktes zu finden. Diese Aufgabe wird durch das erfindungsgemäße Produkt und den erfindungsgemäßen Weg zu seiner Herstellung gelöst.

Es wurde nämlich überraschenderweise gefunden, daß sich entgegen gängigen Vorurteilen und negativ verlaufenen Versuchskompaktierungen dennoch stückiges Calciumformiat mit zufriedenstellenden anwendungstechnischen Eigenschaften erhalten läßt, wenn man das heterodisperse, feinkörnige Material, das typischerweise nach Isolierung und Reinigung als Nebenprodukt eines Verfahrens zur Herstellung von Polyolen, wie beispielsweise Trimethylolethan, Trimethylolpropan oder Pentaerythrit, erhalten wurde, einer Verdichtung, bevorzugt einer Kompaktierung innerhalb eines bestimmten Druckbereiches unterwirft. Bringt man etwa Drücke unterhalb des Druckbereiches, der für das erfindungsgemäße Verfahren kennzeichnend ist, zur Anwendung, so läßt sich stückiges Calciumformiat ohne Zusatz geeigneter Hilfsstoffe lediglich in vergleichsweise fragiler Form herstellen, das beispielsweise äußerst ungünstige Lagereigenschaften, wie erhöhte Bruchinstabilität und gesteigerte Hygroskopie, besitzt. Bringt man Drücke oberhalb des Druckbereiches, der für das erfindungsgemäße Verfahren kennzeichnend ist, zur Anwendung, so leidet hierunter zum einen die Wirtschaftlichkeit, zum anderen erhält man ein außerordentlich verdichtetes Material, das eine deutlich gesteigerte, unerwünschte Bruchstabilität und dadurch bedingt anwendungstechnische Nachteile, wie geringere Auflösungsgeschwindigkeit, größere Härte, erhöhter Energiebedarf bei gegebenenfalls erforderlicher nachgeschalteter Fragmentierung, erhöhte Abrasionseigenschaften etc. aufweist.

Die Erfindung betrifft ein Verfahren zur Herstellung eines stückigen Materials, das Calciumformiat in einer Menge von 5-100 Massen-% und Zusatzstoffe in einer Menge von 95-0 Massen-%, bezogen auf die Gesamtmasse des Materials, enthält und dessen Partikel in einem Korngrößenbereich von 0,2-5 mm liegen, das dadurch gekennzeichnet ist, daß man feinkristallines Calciumformiat einer durchschnittlichen Korngröße unter 0,2 mm und Zusatzstoffe einer Verdichtung unterwirft und, falls erforderlich, das verdichtete Material zur Einstellung der Partikelgröße einer Zerkleinerung oder Fraktionierung oder einer Kombination aus beiden unterwirft.

Die Erfindung betrifft weiter stückiges Material, das Calciumformiat in einer Menge von 5-100 Massen-% und Zusatzstoffe in einer Menge von 95-0 Massen-%, bezogen auf die Gesamtmasse des Materials, enthält und dessen Partikel in einem Korngrößenbereich von 0,2-5 mm liegen.

Einsatzmaterial für das erfindungsgemäße Verfahren ist feinkörniges, pulverförmiges Calciumformiat mit durchschnittlicher Korngröße unter 0,2 mm.

Das erfindungsgemäß herstellbare stückige Calciumformiat bzw. das stückige Material läßt sich erfindungsgemäß beispielsweise dadurch erhalten, daß man das heterodisperse, feinkörnige Material, das typischerweise nach Isolierung und Reinigung als Nebenprodukt eines Verfahrens zur Herstellung von Polyolen, wie beispielsweise Trimethylolethan, Trimethylolpropan oder Pentaerythrit, gewonnen wurde, einer Verdichtung, bevorzugt einer Kompaktierung innerhalb des unten genannten Druckbereiches unterwirft. Grundsätzlich läßt sich hierfür auch Calciumformiat, das auf einem anderen Wege hergestellt und isoliert wurde, einsetzen.

Die Verdichtung zum erfindungsgemäßen stückigen Calciumformiat bzw. zum stückigen Material kann erfindungsgemäß durch dem Fachmann im Grundsatz bekannte Techniken wie Kompaktieren, Pelletieren, Granulieren, Sintern oder Pressen erfolgen. Bevorzugt wird die Technik des Kompaktierens angewendet.

Die Kompaktierung erfolgt mit dem Fachmann bekannten Geräten, bevorzugt mit solchen, die im Handel erhältlich sind. Die Kompaktierung erfolgt im allgemeinen unter den nachfolgend beschriebenen erfindungsgemäßen Bedingungen: Der Anpreßdruck der Walzen beträgt 5-100 kN/cm, bevorzugt 10-70 kN/cm, besonders bevorzugt 15-50 kN/cm. Die Temperatur beträgt 5-80°C, bevorzugt 10-40°C, besonders bevorzugt 15-30°C. Die Umlaufgeschwindigkeit der Walze beträgt 1-100 cm/sec, bevorzugt 3-60 cm/sec, besonders bevorzugt 5-40 cm/sec.

Der Eintrag des pulverförmigen bzw. feinkristallinen Calciumformiates in die Kompaktiervorrichtung kann dabei, gegebenenfalls unter Zusatz weiterer Hilfsstoffe und/oder Komponenten, grundsätzlich mit Hilfe aller dem Fachmann bekannten Förderaggregaten geschehen. Beispielhaft seien Förderbänder, Schnecken und Vibrationsrinnen genannt.

Gegebenenfalls kann mit Hilfe weiterer dem Fachmann bekannter Aggregate eine Vorverdichtung durchgeführt werden, so daß in der Konsequenz hieraus eine mehrstufige Verdichtungssequenz resultiert. Eine derartige Vorverdichtung kann beispielsweise mit Hilfe von Rollenpressen, Vakuumpressen oder Verdichtungsschnecken erzielt werden.

Nach erfindungsgemäßer Durchführung des Verdichtungsschrittes zur Herstellung des erfindungsgemäßen stückigen Calciumformiats bzw. Materials kann angestrebt werden, eine weitgehend einheitliche Partikelgröße des Produktes einzustellen. Hierzu erfolgt eine gezielte Zerstörung von Calciumformiatstücken bzw. Materialstücken, die hinsichtlich ihres Durchmessers oberhalb einer zu wählenden Grenze liegen. Der genannte gezielte Zerstörungsschritt erfolgt beispielsweise mit Hilfe der dem Fachmann bekannten Technik des Schrotens. Im Anschluß an diesen Verfahrensschritt, bei Wahl geeigneter, dem Fachmann bekannter Apparate auch in Kombination mit diesem Verfahrensschritt, wird eine Zerkleinerung bei gleichzeitiger Abtrennung hinreichend kleiner Partikel über eine hinsichtlich ihrer Ausschlußgrenze variable Siebvorrichtung durchgeführt. Die Zerkleinerung kann auf einfache, dem Fachmann bekannte Weise erfolgen. Sie kann beispielsweise mit Hilfe von Siebgranulatoren, Stachelwalzen oder Mehrfachwalzen mit Friktion durchgeführt werden. Die nachfolgende Fraktionierung geschieht beispielsweise unter Einsatz marktüblicher Siebanlagen.

Grundsätzlich ist es auch möglich und ist daher integraler Bestandteil der Erfindung, bei der erfindungsgemäßen Herstellung des stückigen Calciumformiats einen oder mehrere weitere Stoffe zuzusetzen, die gegebenenfalls den Vorgang der Verdichtung und/oder die anwendungstechnischen Eigenschaften und/oder die weitere Verwendung des erfindungsgemäßen Produktes fördern. In bevorzugter Weise erfolgt dabei die Vermischung des eingesetzten feinkörnigen Calciumformiats mit diesen weiteren Stoffen vor der weiteren Verdichtung. Hierdurch gelangt man zu den oben beschriebenen stückigen Materialien mit einem von 100 Massen-% abweichenden Gehalt an Calciumformiat.

Innerhalb der erfindungsgemäßen stückigen, Calciumformiat enthaltenden Materialien beträgt für den Fall, daß ihm im Verlaufe seiner erfindungsgemäßen Herstellung ein oder mehrere Zusatzstoffe beigemischt wurden, der Anteil an reinem Calciumformiat von 5 bis 100 Massen-%, bevorzugt von 25 bis 100 Massen-%, besonders bevorzugt von 50 bis 100 Massen-% und insbesondere bevorzugt von 75 bis 100 Massen-%. Die zu 100 Massen-% fehlenden Anteile sind sodann die Massen der Zusatzstoffe. Calciumformiat und Zusatzstoffe addieren sich zur Gesamtmasse der stückigen Materialien.

Derartige Zusatzstoffe können beispielsweise flüssige oder feste Hilfsmittel sein, die etwa die Kompaktierung des zu verdichtenden Calciumformiates günstig beeinflussen. Insbesondere sollen hierbei solche Stoffe genannt sein, die dem Fachmann als Binde-, Kompaktier- und Formulierhilfsmittel bekannt sind. Als derartige Hilfsmittel seien beispielhaft Wasser, Mineralsäuren ein-, zwei-, drei- und mehrwertige Alkohole, wie insbesondere Glycerin, Carbonsäuren, Carbonsäureester, Carbonsäureamide, Sulfoxide, Fette, Fettsäuren, Fettalkohole, Wachse, Talge und Öle synthetischer oder natürlicher Herkunft genannt. Weiterhin seien als mögliche Hilfsmittel Mono-, Di-, Oligo- und Polysaccharide, wie beispielsweise Glucose, Saccharose, Lactose, Dextrine, Melasse, Stärke und Cellulose, gegebenenfalls außer in der reinen Form in Form von technischen Zubereitungen oder Rohproduktform, genannt.

Stoffe, die gegebenenfalls die anwendungstechnischen Eigenschaften und/oder die weitere Verwendung des erfindungsgemäßen stückigen Calciumformiates positiv beeinflussen können, sind etwa Duft- und Aromastoffe, Kohlenstoff, wie etwa Aktiv- oder Medizinalkohle, Konservierungsstoffe sowie diverse anorganische oder organische Salze natürlicher oder synthetischer Herkunft. Beispielhaft genannt seien Natrium-, Kalium-, Ammonium-, Magnesium-, Calcium- und Eisensalze, etwa als Chloride, Sulfate, Phosphate, Silikate, Carbonate, Hydrogencarbonate, Acetate, Benzoate und Citrate. Weitere Zumischpartner, die die Verwendung des erfindungsgemäßen Produktes positiv beeinflussen, können beispielsweise, vor allem im Bereich der Tierernährung, leistungsfördernde und/oder nutritiv wirksame Substanzen sein. Beispielhaft genannt seien spezielle Futterbestandteile, Vitamine und Pharmazeutika im weitesten Sinne.

Solche Zusatzstoffe, die die anwendungstechnischen Eigenschaften und/oder die weitere Verwendung des stückigen Materials positiv beeinflussen, können auch Eigenschaften von Binde-, Kompaktier- und Formulierungsmitteln aufweisen, so daß letztere nicht mehr erforderlich sind.

Die Vermischung des erfindungsgemäß zum Einsatz kommenden feinkristallinen Calciumformiates mit den genannten weiteren Stoffen kann auf praktisch alle dafür geeignete und dem Fachmann bekannte Weisen erfolgen. Beispielsweise kann die Vermischung in gerührten Behältern, in Schnecken, durch pneumatische Vermischung (pneumatische Verwirbelung), durch Vermahlung in allen dafür geeigneten Mühlentypen, durch vermischendes Absieben, durch gleichmäßige Benetzung oder durch benetzende Agglomeration erfolgen. Grundsätzlich lassen sich auch mehrere der genannten Stoffe mit dem erfindungsgemäß zum Einsatz kommenden Calciumformiat vermischen und der anschließenden Verdichtung und Überführung in das erfindungsgemäße stückige Calciumformiat enthaltende Material unterwerfen.

Die genannten Zusatzstoffe und das erfindungsgemäß als Ausgangsmaterial zum Einsatz kommende feinkörnige Calciumformiat lassen sich in den weiten obengenannten Verhältnissen miteinander vermischen, wobei sich lediglich durch die physikalische Konsistenz der erhaltenen Gemische - sie müssen unter den bei der nachfolgenden Verdichtung sowie der gegebenenfalls erfolgenden weiteren Zerkleinerung und Absiebung zwecks Einstellung der angestrebten Partikelgrößenverteilung fest sein - Limitierungen ergeben.

### Beispiel 1

Ein Kompaktierer des Types BEPEX CS 25 mit geriffelten Glattwalzen (Durchmesser 22,8 cm, Breite 6,4 cm, Tiefe der Riffelung 0,3 mm) und zylindrisch-konischer Schnecke mit zyklischem Konus 160 (Hydraulikdruck 170 bar, Blasenspeicher 110 bar) wurde unter den nachfolgend angegebenen Bedingungen mit gereinigtem und getrocknetem kristallinen Calciumformiat (Schüttdichte 1,111 g/ml), beschickt:
- Druck: 230-240 kN
- Walzengeschwindigkeit: 15 UpM = ca. 18 cm/sec
- Schneckengeschwindigkeit: 30 Upm
- Feedstrom: 310 kg/h

Das durch Kompaktierung gewonnene stückige Calciumformiat wurde in einen Schroter des Typs ALEXANDER, ausgestattet mit einem Sieb der Maschenweite 1,0 mm, übergeführt. Das dabei anfallende Material wurde zur Abtrennung des Unterkorns einer Absiebung über ein Conflux-Sieb (Kreisschwingsieb, Maschenweite 323 µm) unterworfen. Der Siebrückstand (ca. 30 % der Gesamtmenge) wurde einer Siebanalyse unterworfen. Dabei ergaben sich folgende Werte:

| **Siebmaschenweite (mm)** | **Rückstand (Massen-%)** |
|---|---|
| 1,000 | 0,6 |
| 0,500 | 42,6 |
| 0,400 | 23,3 |
| 0,315 | 21,9 |
| <0,315 | 11,6 |

### Beispiel 2

Durchführung analog Beispiel 1, jedoch unter den nachfolgend angegebenen Bedingungen:
- Druck: 285-297 kN
- Walzengeschwindigkeit: 8 UpM = ca. 9,6 cm/sec
- Schneckengeschwindigkeit: 30 UpM
- Feedstrom: 280 kg/h

Das durch Kompaktierung gewonnene stückige Calciumformiat wurde wie in Beispiel 1 in einen Schroter des Typs ALEXANDER, ausgestattet mit einem Sieb der Maschenweite 1,0 mm, übergeführt. Das dabei anfallende Material wurde zur Abtrennung des Unterkorns einer Absiebung über ein Conflux-Sieb (Kreisschwingsieb, Maschenweite 323 µm) unterworfen. Die Siebanalyse des Siebrückstandes (ca. 30 % der Gesamtmenge) ergab folgende Werte:

| **Siebmaschenweite (mm)** | **Rückstand (Massen-%)** |
|---|---|
| 1,000 | 0,5 |
| 0,500 | 42,5 |
| 0,400 | 22,4 |
| 0,315 | 25,1 |
| <0,315 | 9,5 |

### Beispiel 3

Durchführung analog Beispiel 2, jedoch unter Einsatz eines Calciumformiats (Schüttdichte 1,111 g/ml), das zunächst unter Einsatz eines intensiv wirkenden Mischaggregates (Typ Starmix) mit 1,6 Gew.-% einer 38 %igen wäßrigen Zuckerlösung vermischt wurde.

Das durch Kompaktierung gewonnene stückige Calciumformiat wurde wie in Beispiel 2 in einen Schroter des Typs ALEXANDER, ausgestattet mit einem Sieb der Maschenweite 1,0 mm, übergeführt. Das dabei anfallende Material wurde zur Abtrennung des Unterkorns einer Absiebung über ein Conflux-Sieb (Kreisschwingsieb, Maschenweite 323 µm) unterworfen. Die Siebanalyse des Siebrückstandes (ca. 30 % der Gesamtmenge) ergibt folgende Werte:

| **Siebmaschenweite (mm)** | **Rückstand (Massen-%)** |
|---|---|
| 1,000 | 0,4 |
| 0,500 | 42,0 |
| 0,400 | 22,9 |
| 0,315 | 25,7 |
| <0,315 | 9,0 |

## Patentansprüche

1. Verfahren zur Herstellung eines stückigen Materials, das Calciumformiat in einer Menge von 5-100 Massen-% und Zusatzstoffe in einer Menge von 95-0 Massen-%, bezogen auf die Gesamtmasse des Materials, enthält und dessen Partikel in einem Korngrößenbereich von 0,2-5 mm liegen, dadurch gekennzeichnet, daß man feinkristallines Calciumformiat einer durchschnittlichen Korngröße unter 0,2 mm und Zusatzstoffe einer Verdichtung unterwirft und, falls erforderlich, das verdichtete Material zur Einstellung der Partikelgröße einer Zerkleinerung oder Fraktionierung oder einer Kombination aus beiden unterwirft.

2. Verfahren nach Anspruch 1, dadurch gekennzeichnet, daß die Verdichtung in einer oder in mehreren Stufen durch Kompaktieren, Pelletieren, Granulieren, Sintern oder Pressen, bevorzugt durch Sintern oder Kompaktieren, besonders bevorzugt durch Kompaktieren, erfolgt.

3. Verfahren nach Anspruch 2, dadurch gekennzeichnet, daß die Kompaktierung unter einem Preßdruck von 5 bis 100 kN/cm, bevorzugt von 10 bis 70 kN/cm, besonders bevorzugt von 15 bis 50 kN/cm, und bei einer Temperatur zwischen 5 und 80°C, bevorzugt zwischen 10 und 40°C und besonders bevorzugt zwischen 15 und 30°C, durchgeführt wird.

4. Verfahren nach Ansprüchen 1 bis 3, dadurch gekennzeichnet, daß die der Verdichtung nachgeschaltete Zerkleinerung durch Schroten erfolgt.

5. Verfahren nach Ansprüchen 1 bis 4, dadurch gekennzeichnet, daß die der Verdichtung nachgeschaltete Fraktionierung durch Sieben erfolgt.

6. Verfahren nach Ansprüchen 1 bis 5, dadurch gekennzeichnet, daß das feinkristalline Calciumformiat als Nebenprodukt der technischen Herstellung von Polyolen, bevorzugt bei der Herstellung von Neopentylglykol, Trimethylolethan, Trimethylolpropan und Pentaerythrit gewonnen wurde.

7. Verfahren nach Ansprüchen 1 bis 6, dadurch gekennzeichnet, daß die Zusatzstoffe eines oder mehrere feste oder flüssige Binde-, Kompaktier- und Formulierhilfsmittel sind, bevorzugt solche aus der Gruppe von Wasser, Mineralsäuren, ein-, zwei-, drei- und mehrwertigen Alkoholen, Carbonsäuren, Carbonsäureestern, Carbonsäureamiden, Sulfoxiden, Fetten, Fettsäuren, Fettalkoholen, Wachsen, Talgen, Ölen synthetischer oder natürlicher Herkunft, Mono-, Di-, Oligo- und Polysacchariden, sowohl in reiner Form als auch in technischen Zubereitungen oder in Rohproduktform, oder daß die Zusatzstoffe anwendungsfördernde Stoffe sind, bevorzugt aus der Gruppe von Duft- und Aromastoffen, Kohlenstoff, Aktiv- oder Medizinalkoholen, Konservierungsstoffen, anorganischen oder organischen Salzen natürlicher oder synthetischer Herkunft, bevorzugt Natrium-, Kalium-, Ammonium-, Magnesium-, Calcium- und Eisensalzen in Form der Chloride, Sulfate, Phosphate, Silikate, Carbonate, Hydrogencarbonate, Acetate, Benzoate und Citrate, leistungsfördernden und/oder nutritiv wirksamen Substanzen, beispielsweise Futterbestandteilen, Vitaminen und Pharmazeutika, oder daß die Zusatzstoffe sowohl Binde-, Kompaktier- und Formulierhilfsmittel als auch anwendungsfördernde Stoffe sind.

8. Stückiges Material, das Calciumformiat in einer Menge von 5-100 Massen-% und Zusatzstoffe in einer Menge von 95-0 Massen-%, bezogen auf die Gesamtmasse des Materials, enthält und dessen Partikel in einem Korngrößenbereich von 0,2-5 mm liegen.

9. Stückiges Material nach Anspruch 8, das Calciumformiat in einer Menge von 25-100 Massen-%, bevorzugt 50-100 Massen-%, besonders bevorzugt 75-100 Massen-% und Zusatzstoffe in einer Menge von 75-0 Massen-%, bevorzugt 50-0 Massen-%, besonders bevorzugt 25-0 Massen-%, bezogen auf die Gesamtmasse des Materials enthält.

10. Stückiges Material nach Ansprüchen 8 und 9, dessen Partikel in einem Korngrößenbereich von 0,3-3 mm liegen.
